# EUROPÄISCHE PATENTSCHRIFT

(11) **EP 2 010 950 B1**
(45) Veröffentlichungstag und Bekanntmachung des Hinweises auf die Patenterteilung: **12.08.2015**
(21) Anmeldenummer: 07724426.7
(22) Anmeldetag: 20.04.2007
(51) Int. Cl.: G02B 6/34, G02B 6/293

(54) **MULTIWELLENLÄNGEN-LASERSYSTEM UND VERFAHREN FÜR OPHTALMOLOGISCHE ANWENDUNGEN**
MULTIWAVELENGTH LASER SYSTEM AND METHOD FOR OPHTALMOLOGICAL APPLICATIONS
SYSTÈME LASER À LONGUEURS D'ONDE MULTIPLES ET PROCÉDÉ POUR DES APPLICATIONS OPHTALMOLOGIQUES

(30) Priorität: 25.04.2006 DE 102006019127
(43) Veröffentlichungstag der Anmeldung: 07.01.2009
(73) Patentinhaber: Carl Zeiss Meditec AG, 07745 Jena (DE)
(72) Erfinder: ZIMARE, Diego, 07952 Pausa (DE); DICK, Manfred, 07926 Gefell (DE); WIECHMANN, Martin, 07743 Jena (DE); KALIES, Alexander, 07751 Jena (DE); SCHÜTT, Regina, 07749 Jena (DE)
(74) Vertreter: DTS Patent- und Rechtsanwälte Schnekenbühl und Partner mbB
(86) Internationale Anmeldenummer: PCT/EP2007/003490
(87) Internationale Veröffentlichungsnummer: WO 2007/121943

(56) Entgegenhaltungen:
- EP-A1- 0 980 680
- DE-A1- 4 225 191
- US-B1- 6 258 082
- F.G. Bachmann: "Chances and limitations of high power diode lasers" In: M.S. Zediker (ed.): "Proceedings of SPIE: High-power diode laser technology and applications II", 2004, SPIE, PO BOX 10 BELLINGHAM WA 98227-0010 USA, XP040180836, vol. 5336, pages 95-105,
- J. WOLF, E. WERNER: "Grundlagen der Faserkopplung von Diodenlasern", LTJ, vol. 4, September 2008 (2008-09), pages 48-50, Weinheim
- Thorlabs Inc.: "Tools of the trade - catalogue", 2005, Thorlabs Inc. vol. 17 * page 824 - page 826 * * page 854 - page 857 *

## Beschreibung

Die vorliegende Erfindung betrifft ein Multiwellenlängen-Lasersystem und Verfahren für ophthalmologische Anwendungen, insbesondere bezieht sich die vorliegende Erfindung auf ein Multiwellenlängen-Lasersystem, welches mindestens zwei Halbleiterdiodenlaser und ein Lichtleitsystem umfasst.

In der Medizin, insbesondere in der Augenheilkunde, Ophthalmologie, werden laserbasierte Behandlungssysteme eingesetzt, um schonende Eingriffe am menschlichen Körper vorzunehmen. Dabei werden für unterschiedliche Therapien am Auge unterschiedliche Laserwellenlängen verwendet. Beispielsweise werden beim Einsatz von Diodenlasern Leistungen im Bereich von ca. 400 mW im langwelligen Spektralbereich bei 690 nm für photodynamische Therapien eingesetzt.

Der Einsatz von Hochleistungs-Diodenlasern wurde in der Ophthalmologie unter anderem für die "Transpupilläre Thermotherapie" (TTT) bekannt, wobei eine Wellenlänge von 810 nm mit einer Leistung von ungefähr 650 mW appliziert wird.

Darüber hinaus wird bei ophthalmologischen Lasergeräten zusätzlich ein Diodenlaser im roten Wellenlängenbereich mit einer nicht schädigenden Leistung von weniger als 1 mW als sogenannter Pilot- oder Justierstrahl eingesetzt, um vor der Applikation der therapeutischen Wirkstrahlung den Fokus der Strahlung anwendungsspezifisch einzurichten.

Aus der WO 91/12641 und der WO 95/142 51 sind unterschiedliche technische Lösungen aus dem nicht-ophthalmologischen Bereich bekannt, wo zwei oder mehr Laserdioden in einzelne Lichtleitfasern eingekoppelt werden.

Eine weit verbreitete ophthalmologische Lasertherapie ist die sogenannte Laserkoagulation der Netzhaut, die beispielsweise zur Behandlung "diabetischer Retinopathie" eingesetzt wird. Für modifizierte Therapien sind dabei insbesondere der grüne, gelbe und rote Spektralbereich interessant, die durch Multiwellenlängen-Systeme auf der Basis von Gaslasern und reinen diodengepumpten und frequenzvervielfachten Festkörper-Lasersystemen abgedeckt werden, wie in der WO 02/21646 A1 und der EP 1184947 beschrieben wird.

Dokument DE 42 25 191 offenbart ein ophtalmologisches Multiwellenlängen-Lasersystem mit zwei Halbleiter-Diodenlasern unterschiedlicher Wellenlängen, die über Freistrahl-Optik überlagert und in eine gemeinsame Applikationsfaser eingekoppelt werden.

Derartige Multiwellensysteme des Standes der Technik weisen allerdings den Nachteil auf, dass sie ein großes Volumen, große Masse, hohe Komplexität und großen Energiebedarf erfordern und gleichzeitig viel Wärme abgeben, wodurch die Kosten für die Herstellung, den Betrieb und die Instandhaltung derartiger Systeme steigen.

Aufgabe der vorliegenden Erfindung ist es daher, eine Vorrichtung und ein Verfahren für ophthalmologische Anwendungen bereitzustellen, welches, vorhergehend erwähnte Nachteile beseitigt und über ein breites Wellenlängenspektrum verfügt beziehungsweise eine therapeutische Applikation durch unterschiedliche Therapieansätze bedienen kann.

Diese Aufgabe wird durch ein ophtalmologisches Multiwellenlängen-Lasersystem nach Anspruch 1 gelöst. Vorteilhafte Weiterbildungen sind in den Unteransprüchen angegeben. Durch die erfindungsgemäße Vorrichtung ergibt sich der Vorteil, dass mindestens zwei Wellenlängen für ophthalmologische Anwendungen und Therapien zur Verfügung gestellt werden können, wobei das Multiwellenlängen-Lasersystem eine hohe Kompaktheit aufweist.

Ein Multiwellenlängen-Lasersystem ist ein Lasersystem, bei welchem ein Wellenlängenspektrum komplett oder partiell emittiert wird, welches beispielsweise einen Bereich vom Infrarot bis zum UV-Bereich abdeckt. Mittels mindestens zweiunterschiedlichen Wellenlängen ist es möglich, das Lasersystem für verschiedene Therapiebereiche, wie Photokoagulation, Photodynamische Therapie (PDT), Transpupilläre Thermotherapie (TTT), photothermische Tumorbehandlung, etc. einzusetzen.

Unter ophthalmologischen Anwendungen versteht man medizinische und kosmetische Anwendungen, welche die Augenheilkunde betreffen, wie etwa die vorhergehend erwähnte Photokoagulation, Photodynamische Therapie (PDT), Transpupilläre Thermotherapie (TTT), photothermische Tumorbehandlung, etc.

Unter einem Halbleiter-Diodenlaser versteht man einen Diodenlaser oder einen Diodenlaser in Verbindung mit zusätzlichen, wellenlängenbeeinflussenden optischen Elementen, dessen aktives Medium aus einem Halbleitermaterial bestehen. Als Halbleitermaterialien kommen dabei hauptsächlich sogenannte Verbindungshalbleiter zum Einsatz, wie beispielsweise sogenannte III-V-Halbleiter, wie Galliumarsenid (GaAs) oder Indiumantimonid (InSb) oder Halbleiter aus der II. und VI. Hauptgruppe des Periodensystems, sogenannte II-VI-Halbleiter, wie Zinkselenid (ZnSe) oder Cadmiumsulfid (CdS). Es sind aber auch andere Kombinationen denkbar, wie GaInAsSb, AlGaAsSb, AlGaInP, AlAs, GaP, InAs, InP, dotierte Granate oder dotierte Vanadate.

Der Arbeitsstrahl ist dabei jener Strahl, welcher von dem Multiwellenlängen-Lasersystem emittiert wird und für die Ausführung der ophthalmologischen Anwendung benötigt wird und die gewünschte Wirkung im Auge erzielt. So wird beispielsweise bei der Trabekulotomie, einer Operationsmethode zur Beseitigung des Glaukoms (Grüner Star), mittels des Arbeitsstrahls des Halbleiter-Diodenlasers oder eines Festkörperlasers ein sogenannter Entlastungsschnitt am Auge durchgeführt, um das Kammerwasser des Auges abzuleiten.

Unter erster und zweiter Wellenlänge λ₁, λ₂ versteht man, dass die beiden Wellenlängen voneinander verschieden sind und so für unterschiedliche ophthalmologische Anwendungen oder verschiedene Ausführungsformen einer Anwendung je nach Anwendungsfall therapeutisch gezielt eingesetzt werden können. Durch die unterschiedlichen Wellenlängen ergibt sich der Vorteil, dass ein Arbeitsstrahl der ersten Wellenlänge beispielsweise für die ophthalmologische Applikation bei bestimmten vorgegebenen Randbedingungen angewandt werden kann, während der zweite Arbeitsstrahl mit zweiter Wellenlänge für die gleiche ophthalmologische Applikation bei anderen Randbedingungen oder an einer anderen Stelle im Auge eingesetzt werden kann.

Erster und mindestens zweiter Halbleiter-Diodenlaser bedeutet, dass mindestens zwei Halbleiter-Diodenlaser, mit oder ohne zusätzlichen optischen Elementen, die die Wellenlänge beinflussen können, verwendet werden, wobei die Wellenlängen der Arbeitsstrahlen der Halbleiter-Diodenlaser voneinander verschieden sind.

In einem weiteren vorteilhaften Ausführungsbeispiel ist es bevorzugt, dass der erste Arbeitsstrahl eine erste Pulsdauer t₁ und der zweite Arbeitsstrahl eine zweite Pulsdauer t₂ aufweist. Dadurch ergibt sich der Vorteil, dass die Arbeitstrahlen unterschiedlich lange auf dem ophthalmologisch zu behandelnden Objekt appliziert werden können.

In einem weiteren vorteilhaften Ausführungsbeispiel der vorliegenden Erfindung ist vorgesehen, dass das Multiwellenlängen-Lasersystem im cw-Modus (continuous wave-Modus) betrieben wird. Dadurch ergibt sich der Vorteil, dass der Arbeitsstrahl des Halbleiter-Diodenlasers gleichmäßig und für eine längere Zeitperiode ophthalmologisch appliziert werden kann.

In einem weiteren vorteilhaften Ausführungsbeispiel der vorliegenden Erfindung ist vorgesehen, dass das Multiwellenlängen-Lasersystem im pw-Modus (pulsed-wave-Modus) betrieben wird, d.h. die Arbeitsstrahlen werden mittels des Multiwellenlänger-Lasersystems gepulst. Der pw-Modus wird beispielsweise durch entsprechende Steuerungssysteme erreicht, die den Arbeitsstrahl immer wieder periodisch nach gewissen Zeitintervallen unterbrechen. Dadurch ergibt sich der Vorteil, dass der Arbeitsstrahl des Halbleiter-Diodenlasers für kurze Zeitabstände ophthalmologisch appliziert werden kann.

In einem weiteren vorteilhaften Ausführungsbeispiel der vorliegenden Erfindung ist vorgesehen, dass ein Halbleiter-Diodenlaser einen Kristall umfasst aus der Gruppe : GaInAsSb, AlGaAsSb, AlAs, GaAs, GaP, InAs, AlGaInP, AlGaInP/GaAs InP, dotierte Granate oder dotierte Vanadate oder ähnliche Verbindungen zur Erzeugung von Halbleiterstrukturen oder eben genannte Halbleiterstrukturen in Verbindung mit zusätzlichen, wellenlängenbeeinflussenden optischen Elementen. Dadurch ergibt sich der Vorteil, dass mit dem Multiwellenlängen-Lasersystem verschiedene Wellenlängenbereiche erfasst werden können, die mit den herkömmlichen Systemen nicht erreicht werden können und innerhalb gewisser Grenzen durch Variationen von Betriebsparametern kontinuierlich abgestimmt werden können, wodurch das Multiwellenlängen-Lasersystem für unterschiedliche therapeutische Bereiche und deren möglichen unterschiedlichen Randbedingungen eingesetzt werden kann. Darüber hinaus verfügen die unterschiedlichen Halbleiter-Diodenlaser bevorzugt über verschiedene Energien und Strahlprofile.

In einem weiteren vorteilhaften Ausführungsbeispiel der vorliegenden Erfindung ist vorgesehen, dass wenigstens einer der Halbleiterlaser durch eine Laseranordnung aus aktivem Festkörpermaterial und zusätzlichen wellenlängenbeeinflussenden optischen Elementen oder durch ein dotiertes wellenleitendes optisches Material, z.B. eine Lichtleitfaser, mit oder ohne zusätzliche wellenlängenbeeinflussende Elemente ersetzt werden kann.

In einem weiteren vorteilhaften Ausführungsbeispiel der vorliegenden Erfindung ist vorgesehen, dass die Wellenlängen λ₁, λ₂ der Arbeitsstrahlen in einem der folgenden Spektralbereiche liegen: 480 nm bis 520 nm, 530 nm bis 550 nm, 560 nm bis 580 nm, 585 nm bis 615 nm, 620 nm bis 670 nm, 685 nm bis 695 nm, 800 nm bis 820 nm, 1040 nm bis 1070 nm. Dadurch ergibt sich der Vorteil, dass mit dem Multiwellenlängen-Lasersystem verschiedene wellenlängenbereiche erfasst werden können, wodurch das Multiwellenlängen-Lasersystem für unterschiedliche therapeutische Bereiche eingesetzt werden kann. Dadurch ist es möglich, wie bereits vorhergehend erwähnt, das Lasersystem selektiv für unterschiedliche Therapiebereiche und die dabei auftretenden unterschiedlichen Randbedingungen, wie Photokoagulation, Photodynamische Therapie (PDT), Transpupilläre Thermotherapie (TTT), photothermische Tumorbehandlung, etc. einzusetzen.

In einem weiteren vorteilhaften Ausführungsbeispiel der vorliegenden Erfindung ist es vorgesehen, dass die Arbeitsstrahlen voneinander unterschiedliche Energien aufweisen. Dadurch ergibt sich der Vorteil, dass das Multiwellenlängen-Lasersystem für unterschiedliche therapeutische Bereiche eingesetzt werden kann, wo unterschiedliche Energien benötigt werden. So ist es beispielsweise denkbar, dass es einen Arbeitsstrahl mit höherer Energie für die Laser-Trabekulotomie (ALT, DLT, LT) und einen Arbeitsstrahl mit davon abweichender, meist niedriger Energie für die selektive Laser-Trabekulotomie (SLT) gibt.

In einem weiteren vorteilhaften Ausführungsbeispiel der vorliegenden Erfindung ist vorgesehen, dass die Energien der Arbeitsstrahlen zwischen 10 *µ*J und 50 J, bevorzugt zwischen 0,5 mJ und 25 J, besonders bevorzugt zwischen 0,5 mJ und 20 J liegen. Dadurch ergibt sich der Vorteil, dass die Arbeitsstrahlen für unterschiedliche ophthalmologische Anwendungen und Therapien anwendungsspezifisch und bezüglich unterschiedlicher Randbedingungen selektiv eingesetzt werden können.

In einem weiteren vorteilhaften Anwendungsbeispiel der vorliegenden Erfindung ist es vorgesehen, dass die Energien der Arbeitsstrahlen zwischen 10 *µ*J und 50 J, bevorzugt zwischen 0,5 mJ und 25 J, besonders bevorzugt zwischen 0,5 mJ und 20 J liegen und die Wellenlängen aus einem Bereich stammen, welcher die vorhergehend erwähnten Wellenlängenintervalle umfasst. Dadurch ergibt sich der Vorteil, dass die Arbeitsstrahlen für unterschiedliche Ausführungen derselben ophthalmologischen Anwendungen und Therapien eingesetzt werden können, da Arbeitsstrahlen unterschiedlicher Pulsdauer und gleicher Wellenlänge verwendet werden.

In einem weiteren vorteilhaften Anwendungsbeispiel der vorliegenden Erfindung ist es vorgesehen, dass die Energien der Arbeitsstrahlen zwischen 10 *µ*J und 50 J, bevorzugt zwischen 0,5 mJ und 25 J, besonders bevorzugt zwischen 0,5 mJ und 20 J liegen, die Wellenlängen aus einem Bereich stammen, welcher die vorhergehend erwähnten Wellenlängenintervalle umfasst und die Laserquellen aus Kombinationen von aktiven Festkörpermedien mit wellenlängenbeeinflussenden optischen Elementen bestehen. Dadurch ergibt sich der Vorteil, dass die Arbeitsstrahlen für unterschiedliche Ausführungen derselben ophthalmologischen Anwendungen aber unterschiedliche Therapieansätze eingesetzt werden können, da bevorzugt Arbeitsstrahlen unterschiedlicher Pulsdauer-Regime (ms-, *µ*s- oder ns-Regime) verwendet werden können.

In einem weiteren vorteilhaften Ausführungsbeispiel der vorliegenden Erfindung ist vorgesehen, dass ein Strahlparameterprodukt der Halbleiter-Diodenlaser in einem Größenbereich zwischen 0,1 und 125 mm*mrad, bevorzugt zwischen 1 und 100 mm*mrad, besonders bevorzugt zwischen 2 und 80 mm*mrad liegt. Dadurch ergibt sich der Vorteil, dass die Arbeitsstrahlen in eine oder mehrere Lichtleitfasern gekoppelt werden können und für unterschiedliche ophthalmologische Anwendungen und Therapien anwendungsspezifisch eingesetzt werden können, da das Strahlparameterprodukt die Qualität des Laserstrahls mittels seiner Fokussierbarkeit definiert.

In einem weiteren vorteilhaften Ausführungsbeispiel der vorliegenden Erfindung ist vorgesehen, dass mindestens einer der Arbeitsstrahlen eine Pulsdauer zwischen 1 Femtosekunde (1 fs) bis 600 Sekunden, bevorzugt zwischen 1 Picosekunde (1 ps) und 500 Sekunden, besonders bevorzugt zwischen 1 Nanosekunde (1 ns) und 300 Sekunden aufweist. Dadurch ergibt sich der Vorteil, dass das Multiwellenlängen-Lasersystem für nutzerspezifische therapeutische Anwendungen eingesetzt werden kann.

In einem weiteren vorteilhaften Ausführungsbeispiel der vorliegenden Erfindung ist vorgesehen, dass die Arbeitsstrahlen der Halbleiter-Diodenlaser oder der Halbleiter-Diodenlasern in Verbindung mit zusätzlichen, wellenlängenbeeinflussenden optische Elementen oder die Laserquellen aus Kombinationen von aktiven Festkörpermedien mit wellenlängenbeeinflussenden optischen Elementen getrennt voneinander steuerbar sind. Unter getrennt steuerbar versteht man, dass die Halbleiter-Diodenlaser durch eine Benutzerschnittstelle, sowie eine entsprechende externe Steuerung so angesteuert werden, dass es möglich ist, abwechselnd nur den einen Halbleiter-Diodenlaser oder nur den anderen Halbleiter-Diodenlaser anzusteuern, wobei dann von dem entsprechenden Halbleiter-Diodenlaser ein Arbeitsstrahl emittiert wird. Selbstverständlich ist es auch möglich beide Halbleiter-Diodenlaser gleichzeitig anzusteuern und gleichzeitig entsprechende Arbeitsstrahlen unterschiedlicher Wellenlänge zu emittieren. Darüber hinaus können die Arbeitsstrahlen der Halbleiter-Diodenlaser auch so gesteuert werden, dass sich ihre Arbeitsstrahlen in einer Ebene räumlich, beispielsweise mittels eines Scanners, versetzen lassen. Durch die getrennte Ansteuerung ergibt sich der Vorteil, dass die Arbeitsstrahlen anwendungsspezifisch für die unterschiedlichen ophthalmologischen Anwendungen eingesetzt werden können. Beispielsweise können die Arbeitsstrahlen zeitlich versetzt oder gleichzeitig appliziert werden. Ebenso ist es denkbar, dass die Arbeitsstrahlen räumlich versetzt bei der ophthalmologischen Anwendung eingesetzt werden, wobei der erste Arbeitsstrahl der ophthalmologischen Anwendung dient, der räumlich verschobene oder zeitlich versetzte zweite Arbeitsstrahl der Vor- und/oder Nachbehandlung des ophthalmologisch behandelten Gewebes dient. Ebenso ist es denkbar, dass die Arbeitsstrahlen für die ophthalmologische Applikation vorprogrammiert abgestrahlt werden, wobei die Behandlungssequenzen gleichzeitig oder zeitlich getrennt erfolgen.

In einem weiteren vorteilhaften Ausführungsbeispiel der vorliegenden Erfindung ist vorgesehen, dass die von den Halbleiter-Diodenlasern emittierten Arbeitsstrahlen linear polarisiert sind. Dadurch ergibt sich der Vorteil, dass mindestens 2 der Arbeitsstrahlen oder mindestens 1 Arbeitsstrahl von mindestens 2 vorhandenen Arbeitstrahlen und mindestens 1 Hilfsstrahl mechano-optisch überlagert und in ein zusätzlich angeschlossenes Lichtleitleitsystem eingekoppelt werden können. Insbesondere können so die Teilstrahlen derart überlagert werden, dass ihre Strahlparameterprodukte dabei nicht verschlechtert werden

Gemäß einem weiteren Aspekt der Erfindung ist ein Lichtleitsystem vorgesehen, welches mindestens ein faseroptisches System umfasst, wobei die Arbeitsstrahlen eines Multiwellenlängen-Lasersystems in das Lichtleitsystem über das faseroptische System einkoppelbar sind. Dadurch ergibt sich der Vorteil, dass der Strahlengang der Arbeitsstrahlen nicht frei liegt, wie beispielsweise bei einem mechanisch-optischen System, und dadurch das gesamte optische System weniger störanfällig ist. So können beispielsweise Umgebungseinflüsse, wie Staub oder Feuchtigkeit, mit den optischen Bauelementen wechselwirken, was die Gesamtperformance des Systems beeinflusst und einen hohen Aufwand an regelmäßigen Überprüfungen, Neukalibrierungen, etc. verursacht.

Unter einem Lichtleitsystem versteht man dabei eine Vorrichtung, beispielsweise eine optische Faser, ein faseroptisches System oder eine Anordnung optischer Elemente, welche eine Propagation elektromagnetischer Wellen, insbesondere Licht vom IR-Bereich bis zum UV-Bereich, von einem Ende der Vorrichtung zum anderen Ende der Vorrichtung erlaubt.

Ein faseroptisches System ist dabei ein optisches System, welches mindestens eine optische Faser umfasst, wobei dadurch der Strahlengang des Lichts nicht mehr frei liegt, wie im Beispiel mechanisch-optischer Systeme. Unter einem mechanisch-optischen System versteht man dabei ein optisches System, bei welchem die Strahlengänge freiliegen und beispielsweise nicht in einer optischen Faser aufgenommen sind, wodurch sich zahlreiche Nachteile aufgrund von Umwelteinflüssen auf das optische System ergeben. Diese Nachteile werden mittels des durch die Erfindung bereitgestellten faseroptischen Systems behoben.

Unter der Bezeichnung "einkoppelbar" versteht man, dass die Arbeitsstrahlen der Halbleiter-Diodenlaser derart in die optische Faser des Lichtleitsystems gebündelt werden, dass sie sich innerhalb der optischen Faser ohne größere Intensitätsverluste ausbreiten können. Ziel ist dabei, die Arbeitsstrahlen derart in das Lichtleitsystem einzukoppeln, dass es zu keinen nennenswerten Verlusten in der Energie der Arbeitsstrahlen kommt.

In einem weiteren vorteilhaften Ausführungsbeispiel der vorliegenden Erfindung ist vorgesehen, dass das faseroptische System, mindestens eine optische Faser umfasst. Dadurch ergibt sich der Vorteil, dass die Arbeitsstrahlen optimiert und kostengünstig über das faseroptische System übertragen werden können.

In einem weiteren vorteilhaften Ausführungsbeispiel der vorliegenden Erfindung ist vorgesehen, dass das faseroptische System, mindestens zwei optische Fasern umfassen kann die zu einer optischen Faser gekoppelt werden. Dadurch ergibt sich der Vorteil, dass die Arbeitsstrahlen unterschiedlicher Strahlungsquellen, z.B. Halbleiter-Diodenlaser, in einem Faserausgang überlagert bzw. vereinigt werden können.

In einem weiteren vorteilhaften Ausführungsbeispiel der vorliegenden Erfindung ist vorgesehen, dass ein Strahlparameterprodukt mindestens einer Faser des faseroptischen Systems in einem Größenbereich zwischen 0,1 und 125 mm*mrad, bevorzugt zwischen 1 und 100 mm*mrad, besonders bevorzugt zwischen 2 und 80 mm*mrad liegt. Dadurch ergibt sich der Vorteil, dass die Arbeitsstrahlen aus mehreren Strahlquellen, z.B. Halbleiter-Diodenlasern, in eine oder mehrere Lichtleitfasern gekoppelt werden können und für unterschiedliche ophthalmologische Anwendungen und Therapien anwendungsspezifisch eingesetzt werden können, da das Strahlparameterprodukt die Qualität des Laserstrahls mittels seiner Fokussierbarkeit definiert.

In einem weiteren vorteilhaften Ausführungsbeispiel der vorliegenden Erfindung ist vorgesehen, dass die Arbeitsstrahlen an beliebigen Stellen des faseroptischen Systems einkoppelbar sind. Dadurch ergibt sich der Vorteil, dass das Lichtleitsystem anwendungsspezifisch für die jeweilige ophthalmologische Lasertherapie angepasst werden kann. So lässt sich beispielsweise auch ein zusätzlicher Pilotstrahl an einer beliebigen Stelle der optischen Faser einkoppeln um die ophthalmologische Applikation zu unterstützen. Ebenso lassen sich umgekehrt ein oder mehrere Arbeitsstrahlen aus dem Lichtleitsystem auskoppeln, um beispielsweise deren Leistung bei einem Leistungsdetektor zu messen.

In einem weiteren vorteilhaften Ausführungsbeispiel der vorliegenden Erfindung ist vorgesehen, dass die Arbeitsstrahlen an mindestens einer beliebigen Stelle des faseroptischen Systems partiell auskoppelbar sind. Dadurch lassen sich ein oder mehrere Arbeitsstrahlen aus dem Lichtleitsystem auskoppeln, um beispielsweise deren Leistung an einem Leistungsdetektor zu detektieren, zu überwachen bzw. über einen Regelkreis zu steuern.

In einem weiteren vorteilhaften Ausführungsbeispiel der vorliegenden Erfindung ist vorgesehen, dass mindestens eine Faser des faseroptischen Systems einen Durchmesser zwischen 5 µm und 500 µm, bevorzugt zwischen 20 µm und 250 µm, besonders bevorzugt zwischen 50 µm und 100 µm aufweist. Dadurch ergibt sich der Vorteil, dass Arbeitsstrahlen mit entsprechender Energie und benötigtem Strahlparameterprodukt übertragen werden können.

In einem weiteren vorteilhaften Ausführungsbeispiel der vorliegenden Erfindung ist vorgesehen, dass das optische Fasersystem an seinem distalen Ende mindestens eine Einzelkernfaser ist, die mindestens einen Faserkern mit einem Kerndurchmesser zwischen 10 µm und 600 µm, bevorzugt zwischen 30 µm und 400 µm, besonders bevorzugt zwischen 40 µm und 160 µm aufweist. Dadurch ergibt sich der Vorteil, dass Arbeitsstrahlen mit entsprechender Energie und geeignetem Strahlparameterprodukt zum Applikationsort übertragen werden können.

In einem weiteren vorteilhaften Ausführungsbeispiel der vorliegenden Erfindung ist vorgesehen, dass an den proximalen Enden und am distalen Ende von mindestens einer Faser des faseroptischen Systems die numerische Apertur (N.A.) des Faserkerns zwischen 0,01 und 0,4, bevorzugt zwischen 0,05 und 0,22, besonders bevorzugt zwischen 0,07 und 0,15 beträgt. Dadurch ergibt sich der Vorteil, dass mit dem Lichtleitsystem Strahlung effizient für ophthalmologische Anwendungen und ihre optischen Erfordernisse übertragen werden kann.

In einem weiteren vorteilhaften Ausführungsbeispiel der vorliegenden Erfindung ist vorgesehen, dass das faseroptische System mit einem mechanisch-optischen System gekoppelt ist, um eine Anpassung der Strahlparameter der Laserquelle, z.B. Halbleiterlaser und das faseroptische System, zu ermöglichen Dadurch lässt sich die Energie verlust-minimiert von der Laserquelle zum Ort der Applikation transportieren.

In einem weiteren vorteilhaften Ausführungsbeispiel der vorliegenden Erfindung ist vorgesehen, dass mindestens eine Faser des faseroptischen Systems eine Dotierung aufweist. Dadurch ergibt sich der Vorteil, dass mindestens einer der Arbeitsstrahlen aus den bereits genannten Wellenlängenbereichen direkt im faseroptischen System erzeugt, wellenlängengewandelt oder verstärkt wird. Dadurch ergibt sich der Vorteil, dass die Komplexität des gesamten Systems reduziert werden kann.

In einem weiteren vorteilhaften Ausführungsbeispiel der vorliegenden Erfindung ist vorgesehen, dass mindestens eine Faser des faseroptischen Systems und eine Dotierung aufweist als Doppelkernfaser ausgeführt werden kann. Dadurch ergibt sich der Vorteil, dass Verluste bei der Einkopplung der Laserquellen in das faseroptische System minimiert werden können.

In einem weiteren vorteilhaften Ausführungsbeispiel der vorliegenden Erfindung ist vorgesehen, dass ein Pilotstrahl, welcher aus einer Halbleiter-Laserdiode oder aus einer Halbleiter-Leuchtdiode emittiert wird, in das Lichtleitsystem einkoppelbar ist. Dadurch ergibt sich der Vorteil, dass die Arbeitsstrahlen des Multiwellenlängen-Lasersystems in Kombination mit dem Lichtleitsystem gezielt ophthalmologisch appliziert werden können, da die Arbeitsstrahlen für den Anwender vor oder während der Anwendung nicht sichtbar sind und somit die genaue Lage der Arbeitsstrahlen vor und während der Applikation sichtbar gemacht wird, was die Sicherheit des Systems gewährleistet.

In einem weiteren vorteilhaften Ausführungsbeispiel der vorliegenden Erfindung ist vorgesehen, dass der Pilotstrahl eine Leistung von weniger als 1 mW, bevorzugt von weniger als 0,1 mW aufweist. Dadurch ergibt sich der Vorteil, dass es durch den Pilotstrahl zu keiner Schädigung des ophthalmologisch behandelten Gewebes kommt, da der Pilotstrahl lediglich unterstützend bei der ophthalmologischen Lasertherapie eingesetzt wird und der Festlegung des später durch die Arbeitsstrahlen ophthalmologisch behandelten Bereichs dient.

In einem weiteren vorteilhaften Ausführungsbeispiel der vorliegenden Erfindung ist vorgesehen, dass an eine oder mehrere Fasern des faseroptischen Systems eine Applikationsfaser anschließbar ist. Je nach ophthalmologischer Applikation kann die Applikationsfaser beispielsweise direkt in das Auge eingeführt werden oder Bestandteil des Lichtleitsystems sein. Anschließend kann mit dem durch das Multiwellenlängen-Lasersystem unterstützten Therapieverfahren begonnen werden. Durch Verwendung einer Applikationfaser ergibt sich der Vorteil, dass das Lichtleitsystem für ophthalmologische Anwendungen anwendungsspezifisch optimiert werden kann, und die Arbeitsstrahlen des Multiwellenlängen-Lasersystems präziser bei dem ophthalmologisch zu behandelndem Objekt zum Einsatz gebracht werden können.

In einem weiteren vorteilhaften Ausführungsbeispiel der vorliegenden Erfindung ist vorgesehen, dass das Strahlparameterprodukt des faseroptischen Systems kleiner oder gleich dem Strahlparameterprodukt der Applikationsfaser ist. Dadurch ergibt sich der Vorteil, dass die das faseroptische System durchdringenden Arbeitsstrahlen optimal in die Applikationsfaser eingekoppelt werden können.

In einem weiteren vorteilhaften Ausführungsbeispiel der vorliegenden Erfindung ist vorgesehen, dass das faseroptische System am distalen Ende aus mehr als einer Faser besteht und dass das faseroptische System eine faseroptische oder mechanisch-optische Komponente enthält mit der die eingekoppelten Arbeitsstrahlen zwischen Ausgängen des faseroptischen Systems umgeschaltet bzw. verteilt werden können.

In einem weiteren vorteilhaften Ausführungsbeispiel der vorliegenden Erfindung ist vorgesehen, dass das faseroptische System und das Lichtleitsystem mindestens eine polarisationserhaltende optische Faser umfassen. Dadurch ergibt sich der Vorteil, dass die für unterschiedliche therapeutische Anwendungen gedachten Arbeitsstrahlen in das Lichtleitsystem eingekoppelt und dort polarisationsabhängig überlagert werden können, ohne dass es zu wesentlichen Verlusten in der Energien und Leistungen der Arbeitsstrahlen kommt.

In einem weiteren vorteilhaften Ausführungsbeispiel der vorliegenden Erfindung ist vorgesehen, dass das faseroptische System mindestens ein mechanisch-optisches System umfasst. Unter einem mechanisch-optischem System versteht man ein optisches System, bei welchem die Strahlengänge freiliegen und beispielsweise nicht in einer optischen Faser aufgenommen sind, wodurch sich zahlreiche Nachteile aufgrund von Umwelteinflüssen auf das optische System ergeben, allerdings ergibt sich durch das mechanisch-optische System auch der Vorteil, dass das Lichtleitsystem individuell an spezifische ophthalmologische Anwendungen angepasst werden kann und somit die Flexibilität des Lichtleitsystems erhöht wird.

In einem weiteren vorteilhaften Ausführungsbeispiel der vorliegenden Erfindung ist vorgesehen, dass das Lichtleitsystem mindestens einen dichroitischen Koppler umfasst. Unter einem dichroitischen Koppler versteht man eine Vorrichtung, dessen Optik Licht eines genau definierten Wellenlängenbereichs transmittiert und Licht eines anderen genau definierten Wellenlängenbereichs reflektiert. Dadurch ergibt sich der Vorteil, dass die Arbeitsstrahlen in dem Lichtleitsystem überlagert werden können.

In einem weiteren vorteilhaften Ausführungsbeispiel der vorliegenden Erfindung ist vorgesehen, dass das Lasersystem mindestens eine Leistungssteuerung und Leistungsüberwachung umfasst. Unter Lasersystem versteht man hier im Weiteren die Gesamtheit aller Komponenten des Multiwellenlängen-Lasersystems und des Lichtleitsystems. Die Leistungsüberwachung dient dazu, die Leistung der von dem Multiwellenlängen-Lasersystem in das Lichtleitsystem eingekoppelten Arbeitsstrahlen an einer bestimmten Stelle der optischen Faser zu überwachen, um zu gewährleisten, dass die Arbeitsstrahlen am Ende der optischen Faser eine entsprechend Energie und Leistung aufweisen, die später über die Applikationsfaser bei der ophthalmologischen Anwendung appliziert wird. Sollte die tatsächliche Energie und Leistung von der gewünschten und für die Therapie notwendigen Energie und Leistung abweichen, wird durch die Leistungssteuerung bewirkt, dass die HalbleiterDiodenlaser derart rückkoppelbar angesteuert werden, dass sich die für die ophthalmologische Anwendung notwendige Energie und Leistung in der optischen Faser und am Faserende ergeben. Dadurch ergibt sich der Vorteil, dass das Lichtleitsystem mit dem Multiwellenlängen-Lasersystem anwendungsspezifisch für ophthalmologische Anwendungen eingestellt werden kann und dass.das Lichtleitsystem und die von dem Multiwellenlängen-Lasersystem in das Lichtleitsystem eingekoppelten Arbeitsstrahlen laufend überwacht werden können und eventuell notwendige Parameterveränderungen am System vorgenommen werden können.

In einem weiteren vorteilhaften Ausführungsbeispiel der vorliegenden Erfindung ist vorgesehen, dass das Lasersystem mindestens eine Systemsteuerung umfasst. Die Systemsteuerung ist für die vorhergehend erwähnte Leistungsüberwachung und -Steuerung notwendig, um die Halbleiter-Diodenlaser entsprechend anzusteuern, damit die für die Applikation der Lasertherapie notwendigen Energien der Arbeitsstrahlen erreicht werden können. Die Systemsteuerung setzt sich dabei beispielsweise aus Treibern, Sensoren, Überwachungs- und Steuerelektronik, einem elektronischen Rechner und entsprechender Steuerungssoftware zusammen. Dadurch ergibt sich der Vorteil, dass das Lichtleitsystem mit dem Multiwellenlängen-Lasersystem anwendungsspezifisch für ophthalmologische Anwendungen rückkoppelbar eingestellt werden kann.

In einem weiteren vorteilhaften Ausführungsbeispiel der vorliegenden Erfindung ist vorgesehen, dass das Lichtleitsystem mindestens einen optischen Spiegel umfasst. Optische Spiegel sind dabei derart ausgelegt, dass sie das Laserlicht der Arbeitsstrahlen vollständig reflektieren oder nur einen Teil des einfallenden Lichts. Als bevorzugte optische Spiegel werden dabei Spiegel eingesetzt, wie sie üblicherweise bei Strahlengängen mit Halbleiter-Diodenlasern eingesetzt werden. Die optischen Spiegel dienen auch dazu, die Arbeitsstrahlen vorerst mittels einer mechanisch-optischen Systems in das faseroptische System einzukoppeln, falls dies bei entsprechenden örtlichen Gegebenheiten notwendig ist. Durch die Verwendung von optischen Spiegeln ergibt sich der Vorteil, dass das Lichtleitsystem und/oder das Lasersystem für ophthalmologische Anwendungen entsprechend anwendungsspezifisch eingerichtet werden kann.

In einem weiteren vorteilhaften Ausführungsbeispiel der vorliegenden Erfindung ist vorgesehen; dass das Lasersystem ein System zur Auswertung rückgestreuter und/oder partiell reflektierter Arbeitsstrahlen umfasst. Das System zur Auswertung derartiger rückgestreuter und/oder partiell reflektierter Strahlen kann sich dabei derart zusammensetzen, dass die bei der optischen Faser und/oder dem Lichtleitsystem eingekoppelte Energie und Leistung mit der beim Ende der optischen Faser ausgekoppelten Energie und Leistung verglichen wird. Befindet sich das Verhältnis zwischen eingekoppelter Energie und Leistung und ausgekoppelter Energie und Leistung in einem gewissen empirisch ermittelten Intervall, so nimmt das System keine entsprechende Korrektur über die vorhergehend erwähnte Systemsteuerung vor. Weicht allerdings das Verhältnis zu sehr von den entsprechenden Werten ab, wird das Lichtleitsystem mittels des Systems zur Auswertung der rückgestreuten und/oder partiell reflektierten Arbeitsstrahlen solange korrigiert, bis das gewünschte Verhältnis erreicht ist. Durch die Verwendung eines Systems zur Auswertung rückgestreuter und/oder partiell reflektierter Arbeitsstrahlen ergibt sich der Vorteil, dass die aus dem Multiwellenlängen-Lasersystem in das Lichtleitsystem eingekoppelten Arbeitsstrahlen laufend überwacht werden können und eventuell notwendige Parameterveränderungen am System vorgenommen werden, damit es zu keinen für den Anwender des Lichtleitsystems gefährlichen Zuständen der Arbeitsstrahlen kommt und um die ophthalmologische Anwendung des Systems zu optimieren.

In einem weiteren vorteilhaften Ausführungsbeispiel der vorliegenden Erfindung ist vorgesehen, dass das Lasersystem eine Remote-Steuerung umfasst. Unter einer Remote-Steuerung wird beispielsweise verstanden, dass das Multiwellenlängen-Lasersystem in Verbindung mit dem Lichtleitsystem auch von einem zu dem Applikationsort entfernten Ort mittels eines entsprechenden Endgerätes (Computer, Touch-Pad, Bluetooth-Gerät etc.) überwacht und gesteuert werden. Dadurch ergibt sich der Vorteil, dass das Multiwellenlängen-Lasersystem auch bei weiter entfernt aufgestelltem Applikator bedient und kontrolliert wird.

In einem weiteren vorteilhaften Ausführungsbeispiel der vorliegenden Erfindung ist vorgesehen, dass das Strahlparameterprodukt der Arbeitsstrahlen der Halbleiter-Diodenlaser kleiner oder gleich dem Strahlparameterprodukt des Lichtleitsystems und kleiner als 125 mm*mrad ist. Dadurch ergibt sich der Vorteil, dass die Arbeitsstrahlen optimal in das Lichtleitsystem eingekoppelt werden können und es somit zu einer passenden Ausnutzung des Lichtleitsystems kommt.In einem weiteren vorteilhaften Ausführungsbeispiel der vorliegenden Erfindung ist vorgesehen, dass das Strahlparameterprodukt an den Faserkoppelstellen des Lichtleitsystems vergleichbar mit dem Strahlparameterprodukt der Halbleiter-Diodenlaser ist. Dadurch ergibt sich der Vorteil, dass die Arbeitsstrahlen optimal in das Lichtleitsystem eingekoppelt werden.

In einem weiteren vorteilhaften Ausführungsbeispiel der vorliegenden Erfindung ist vorgesehen, dass das Lasersystem eine Steuerung der Halbleiter-Diodenlaser, eine Nutzer-Schnittstelle und ein Applikationssystem umfasst. Die dabei vorgestellte Steuerung entspricht der vorhergehend beschriebenen Systemsteuerung, die Nutzer-Schnittstelle ist die Schnittstelle zwischen dem Nutzer des Systems und der Systemsteuerung und das Applikationssystem ist die Schnittstelle zwischen dem Halbleiter-Diodensystem und dem Patienten. In dem Applikationssystem können sich beispielsweise das Lichtleitsystem mit der optischen Faser, eine Spaltlampe, Applikationsfaser, etc. befinden. Weiter können die Arbeitsstrahlen in das Applikationssystem mittels zweier optischer Fasern eingekoppelt werden. Dadurch ergibt sich der Vorteil, dass das Lichtleitsystem mit dem Multiwellenlängen-Lasersystem einfach und kostengünstig zu bedienen ist.

In einem weiteren vorteilhaften Ausführungsbeispiel der vorliegenden Erfindung ist vorgesehen, dass das Lichtleitsystem eine Spaltlampe umfasst, die das Laserlicht der mehrerer Wellenlängen der Arbeitsstrahlen bei gleichen geforderten Behandlungsparametern identisch in das Auge abbilden kann. Die Spaltlampe ist ein wichtiges Werkzeug in der Ophthalmologie. Mit ihrer Hilfe kann man genaue Details im vorderen Bereich des Auges sehen. Mit einstellbarer Vergrößerung und spezieller seitlicher Beleuchtung, dem so genannten Lichtspalt, kann man zahlreiche Erkrankungen des Auges, wie beispielsweise Entzündungen, den grauen Star, etc. erkennen. Die Spaltlampe dient während der ophthalmologischen Anwendung dazu, die Behandlung zu kontrollieren und die Laserenergie der verschiedenen Arbeitsstrahlen in das Auge hinein zu applizieren. Dadurch ergibt sich der Vorteil, dass das Lichtleitsystem mittels der Spaltlampe für ophthalmologische Anwendungen, die unterschiedliche Wellenlängen bedingen, optimiert und somit das Therapieverfahren verbessert wird.

In einem weiteren vorteilhaften Ausführungsbeispiel der vorliegenden Erfindung ist vorgesehen, dass die in das Lichtleitsystem einkoppelbaren Arbeitsstrahlen zumindest teilweise auskoppelbar sind, insbesondere in eine Leistungsüberwachung einkoppelbar sind. Dabei ist es bevorzugt, dass die Arbeitsstrahlen mit einem bestimmten Prozentsatz ihrer Intensität, beispielsweise mit 1 %, aus dem Lichtleitsystem ausgekoppelt werden und bevorzugt in eine Leistungsüberwachung eingekoppelt werden können. Durch die teilweise Auskopplung ergibt sich der Vorteil, dass während die Applikation der Arbeitsstrahlen auf einen ophthalmologisch zu behandelnden Bereich nicht unterbrochen zu werden braucht. Eine Leistungsüberwachung ist bevorzugt eine mechanische oder elektronische Vorrichtung zur Ermittlung der Energie und/oder Leistung der von den Multiwellenlängen-Lasersystem emittierten Arbeitsstrahlen.

In einem weiteren vorteilhaften Ausführungsbeispiel der vorliegenden Erfindung ist vorgesehen, dass die Leistung und/oder Energie des ersten Halbleiter-Diodenlasers und/oder des zweiten Halbleiter-Diodenlasers mittels einer Leistungssteuerung über eine Steuerung einstellbar sind. Nachdem von der Leistungsüberwachung die Energie und/oder Leistung der Arbeitsstrahlen ermittelt wurde, wird mittels einer Leistungssteuerung, die bevorzugt mit der Leistungsüberwachung verbunden ist, die Energie und/oder Leistung automatisch oder manuell eingestellt, indem der Ist-Wert mit dem Soll-Wert der Energie und/oder Leistung, die für die ophthalmologische Behandlung benötigt werden, der zu applizierenden Arbeitsstrahlen verglichen wird.

Stimmen Ist- und Soll-Wert nicht überein, so sendet die Leistungssteuerung ein Signal an die Steuerung des Multiwellenlängen-Lasersystems, wobei die Energie und/oder Leistung in Abhängigkeit von der Differenz zwischen Ist- und Soll-Wert um einen gewissen Wert verändert wird. Diese Veränderung wird anschließend von der Leistungsüberwachung registriert und an die Leistungssteuerung übertragen.

Weichen Ist- und Soll-Wert weiterhin von einander ab, so verändert die Leistungssteuerung erneut die Energie und/oder Leistung des Multiwellenlängen-Lasersystems, indem sie ein Signal an die Steuerung des Multiwellenlängen-Lasersystem sendet, um die Leistung und/oder Energie des Multiwellenlängen-Lasersystem um eine bestimmte Veränderung zu verändern. Durch den ständigen Vergleich zwischen Ist- und Sollwert durch die Leistungsüberwachung und durch die ständige Signalübertragung der Leistungssteuerung an die Steuerung des Multiwellenlängen-Lasersystem entsteht eine Art Rückkoppelung bis der benötigte Soll-Wert mit dem momentanen Ist-Wert des Multiwellenlängen-Lasersystems bis zu einer gewissen prozentualen Abweichung miteinander übereinstimmen.

In einem weiteren vorteilhaften Ausführungsbeispiel der vorliegenden Erfindung ist vorgesehen, dass die Halbleiter-Diodenlaser mittels zweier separat angepasster Laseroszillatoren in das Lichtleitsystem einkoppelbar sind. Dabei dient jeweils ein Laseroszillator für die Applikation der Arbeitstrahlen bei der Photokoagulation/ALT im cw-Modus (continuous wave Modus) und der zweite Laseroszillator für die Applikation der Arbeitsstrahlen im pw-Modus (pulsed wave-Modus) bei der Selektiven Trabekulotomie (SLT). Die Laseroszillatoren können beispielsweise eine gemeinsame Steuerung, ein gemeinsames Userinterface und ein gemeinsames Applikationssystem (z.B. eine Spaltlampe) aufweisen. Ebenso kann ein zweiteiliges gemeinsames Applikationssystem eingesetzt werden, welches z.B. aus einer Spaltlampe und einem Linksystem für die SLT besteht. Die Arbeitsstrahlen können dem Applikationssystem beispielsweise über eine bzw. zwei Lichtleitfasern zugeführt werden. Ebenso kann das Multiwellenlängen-Lasersystem bei der Applikation für die SLT direkt an das Applikationssystem angekoppelt werden. Durch die in diesem Ausführungsbeispiel aufgeführten Anordnungsmöglichkeiten ergibt sich der Vorteil, dass Arbeitsstrahlen im cw-Modus oder im pw-Modus mit entsprechender Pulsrate eingekoppelt werden können.

In einem weiteren vorteilhaften Ausführungsbeispiel der vorliegenden Erfindung ist vorgesehen, dass das Lichtleitsystem ein 2-teiliges Applikationssystem umfasst, welches eine Spaltlampe und ein Linksystem für selektive Lasertrabekulotomie (SLT) umfasst, die miteinander mechanisch gekoppelt sind. Die Spaltlampe wird aufgrund der bereits vorhergehenden Ausführungen verwendet. Das Linksystem für Lasertrabekulotomie (SLT) erlaubt die zusätzliche Anwendung des Multiwellenlängen-Lasersystems im pw-Modus und bei einem anderen geforderten Strahlparameterprodukt der Anwendung. Dadurch ergibt sich der Vorteil, dass das Lichtleitsystem auf einfache Art und Weise für unterschiedliche ophthalmologische Anwendungen verwendbar ist.

Die Aufgabe wird auch durch ein Verfahren für ophthalmologische Anwendungen, insbesondere selektiver Laser-Trabekulotomie (SLT) und/oder Laser-Trabekulotomie (ALT, DLT, LT) gelöst, wobei ein Multiwellenlängen-Lasersystem und/oder ein Lichtleitsystem gemäß den vorhergehenden Ausführungen verwendet werden. Dadurch ergibt sich der Vorteil, dass die selektive Laser-Trabekulotomie (SLT) und/oder Laser-Trabekulotomie (ALT, DLT, LT) für entsprechende ophthalmologische Lasertherapien verbessert und kostengünstiger werden.

Das Multiwellenlängen-Lasersystem umfasst dabei bevorzugt Halbleiterdiodenlaser, die einen Halbleiterkristall umfassen, der aus der Gruppe stammt, die umfasst: GaInAsSb, AlGaAsSb, AlAs, GaAs, GaP, InAs, AlGaInP, AlGaInP/GaAs, InP, dotierte Granate oder dotierte Vanadate, YAG, Nd:YAG.

Die Arbeitsstrahlen der Halbleiter-Diodenlaser sind bevorzugt mittels zweier separat angepasster Laseroszillatoren in das Lichtleitsystem einkoppelbar. Dabei dient jeweils ein Laseroszillator für die Applikation der Arbeitstrahlen bei der Photokoagulation/ALT im cw-Modus (continuous wave Modus) und der zweite Laseroszillator für die Applikation der Arbeitsstrahlen im pw-Modus (pulsed wave-Modus) bei der Selektiven Trabekulotomie (SLT).

Die Laseroszillatoren können bevorzugt eine gemeinsame Steuerung, ein gemeinsames Userinterface und ein gemeinsames Applikationssystem (z.B. eine Spaltlampe) aufweisen. Bevorzugt können die Laseroszillatoren, die Multiwellenlängen-Laser, die gemeinsame Steuerung, das gemeinsame Userinterface und das gemeinsame Applikationsgeräte aus einer Geräteeinheit, d.h. einem Gerät bestehen.

Ebenso kann bevorzugt ein zweiteiliges gemeinsames Applikationssystem eingesetzt werden, welches z.B. aus einer Spaltlampe und einem Linksystem für die SLT besteht.

Die Arbeitsstrahlen können dem Applikationssystem beispielsweise über eine bzw. zwei Lichtleitfasern zugeführt werden. Ebenso kann das Multiwellenlängen-Lasersystem bei der Applikation für die SLT direkt an das Applikationssystem angekoppelt werden.

Durch die in diesem Ausführungsbeispiel aufgeführten Anordnungsmöglichkeiten ergibt sich der Vorteil, dass Arbeitsstrahlen im cw-Modus oder im pw-Modus mit entsprechender Pulsrate eingekoppelt werden können.

Im Folgenden sollen weitere vorteilhafte Ausgestaltungen der Erfindung anhand der Zeichnung erläutert werden. Hierbei zeigt
- Fig. 1: eine schematische Darstellung des Multiwellenlängen-Lasersystems und des Lichtleitsystems der vorliegenden Erfindung,
- Fig. 2: die in Fig. 1 dargestellte schematische Ansicht der Erfindung mit zusätzlichem Pilotstrahl und Leistungsüberwachung,
- Fig. 3: die in Fig. 2 dargestellte schematische Ansicht der Erfindung mit einem zusätzlichen Lichtleitsystem inklusive Applikationsfaser,
- Fig. 4: die in Fig. 3 dargestellte schematische Ansicht der Erfindung mit einem zusätzlichem mechanisch-optischen System, Dotierung, Steuerung und Nutzerschnittstelle, die nicht unter die Erfindung fällt.

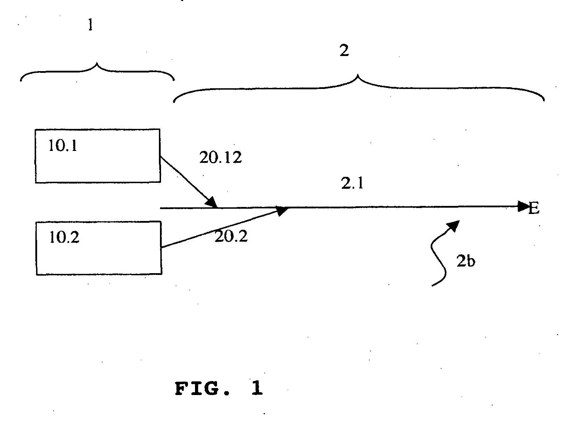

Figur 1 zeigt eine schematische Darstellung des Multiwellenlängen-Lasersystems 1 und des Lichtleitsystems 2 der vorliegenden Erfindung.

Dabei setzt sich das Multiwellenlängen-Lasersystem 1 aus den beiden Halbleiter-Diodenlasern 10.1 und 10.2 zusammen, die Arbeitsstrahlen 20.1 und 20.2 mit Wellenlängen λ₁ und λ₂ emittieren.

Weiter ist in Figur 1 das Lichtleitsystem 2 dargestellt, welches eine optische Faser 2.1 umfasst, und wobei das Lichtleitsystem 2 durch die Verwendung einer optischen Faser 2.1 in dem hier dargestellten Beispiel ein so genanntes faseroptisches System ist.

Die von den Halbleiter-Diodenlasern 10.1 und 10.2 emittierten Arbeitsstrahlen 20.1 und 20.2 werden an gleichen oder unterschiedlichen Stellen der optischen Faser 2.1 in das Lichtleitsystem 2 eingekoppelt und propagieren anschließend durch die optische Faser 2.1 bis sie am Ende E der optischen Faser 2.1, welche aus mindestens einem Faserkern besteht, ausgekoppelt werden und für ophthalmologische Anwendungen bereitstehen.

Die Wellenlänge λ₁ des Arbeitsstrahls 20.1 des ersten Halbleiter-Diodenlasers, welcher in diesem Ausführungsbeispiel ein InGaAlP-Laser ist, beträgt 635 nm, die Wellenlänge λ₂ des Arbeitsstrahls 20.2 des zweiten Halbleiter-Diodenlasers, welcher in diesem Ausführungsbeispiel ein GaAlAs-Laser ist, beträgt 820 nm.

Die Energie des ersten Arbeitsstrahls 20.1 beträgt 0,5 mJ, die Energie des zweiten Arbeitsstrahls 20.2 beträgt 4 J.

Darüber hinaus wird der erste Arbeitsstrahl 20.1 im cw-Modus, der zweite Arbeitsstrahl 20.2 im pw-Modus betrieben, wobei er mittels eines Strahlunterbrechers (Laser-Shutter) in regelmäßigen Intervallen unterbrochen wird.

Mit den vorhergehenden Parametern sind die Arbeitsstrahlen 20.1 und 20.2 für unterschiedliche ophthalmologische Anwendungen, wie beispielsweise Photokoagulation, Photodynamische Therapie (PDT), Transpupilläre Thermotherapie (TTT), photothermische Tumorbehandlung, etc. geeignet.

Weiter liegt das Strahlparameterprodukt der Arbeitsstrahlen 20.1 und 20.2 der Halbleiter-Diodenlaser 10.1, 10.2 bei 11 mm*mrad.

Weiter sind die Arbeitsstrahlen 20.1, 20.2 der Halbleiter-Diodenlaser 10.1, 10.2 des Multiwellenlängen-Lasersystems getrennt voneinander steuerbar, dass bedeutet, dass deren Emission zeitlich versetzt erfolgt. Wobei der zuerst am ophthalmologisch zu behandelnden Objekt auftreffende Arbeitsstrahl 20.1 eine erste ophthalmologische Behandlung durchführt und der zweite zeitlich versetzt Arbeitsstrahl 20.2 eine entsprechende ophthalmologische Nachbehandlung durchführt.

Darüber hinaus sind die von den Halbleiter-Diodenlasern 10.1, 10.2 emittierten Arbeitsstrahlen 20.1, 20.2 linear polarisiert, um in das Lichtleitsystem 2 leichter einkoppelbar zu sein.

Die optische Faser 2.1 weist bevorzugt einen Durchmesser von 100 µm auf.

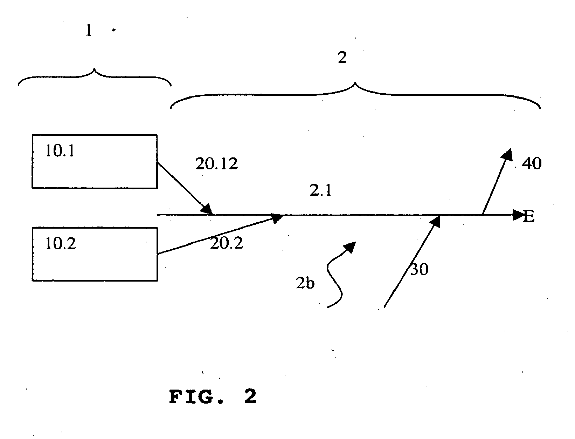

In Figur 2 ist eine weitere bevorzugte Ausführungsform des Multiwellenlängen-Lasersystems 1 und des Lichtleitsystems 2 dargestellt.

Zusätzlich zu dem in Figur 1 beschriebenen Multiwellenlängen-Lasersystem 1 und Lichtleitsystem 2 weist die erfindungsgemäße Vorrichtung am faseroptischen System mindestens einen weiteren Eingang, z.B. für einen Pilotstrahl 30 oder einen weiteren Arbeitsstrahl und mindestens einen weiteren Ausgang mit partieller Auskopplung von 0 - 100 Prozent für z. B. eine Leistungsüberwachung 40 auf.

Bei der in Figur 2 dargestellten Vorrichtung werden ebenso Arbeitsstrahlen 20.1 und 20.2 in die optische Faser 2.1 des Lichtleitsystems 2 eingekoppelt und durchlaufen die optische Faser 2.1 bis sie am Ende E dieser optischen Faser 2.1 aus dem Lichtleitsystem 2 wieder auskoppeln und für ophthalmologische Anwendungen zur Verfügung stehen.

Zur operativen Unterstützung bei ophthalmologischen Anwendungen wird ein Pilotstrahl 30 eingekoppelt, welcher eine Wellenlänge von 600 nm bis 670 nm und eine Leistung von kleiner 0,5 mW aufweist. Der Pilotstrahl ist dabei vorzugsweise eine auf Halbleitertechnologie basierende Lichtquelle, z.B. ein Halbleiter-Diodenlaser.

Durch den Pilotstrahl 30 ist es einem Nutzer des Multiwellenlängen-Lasersystems und des Lichtleitsystems möglich, die spätere Lage der Arbeitsstrahlen 20.1 und 20.2 auf dem opthalomologisch zu behandelnden Bereich des Auges vorher anzupeilen und so das Lasersystem noch sicherer für ophthalmologische Anwendungen zu machen.

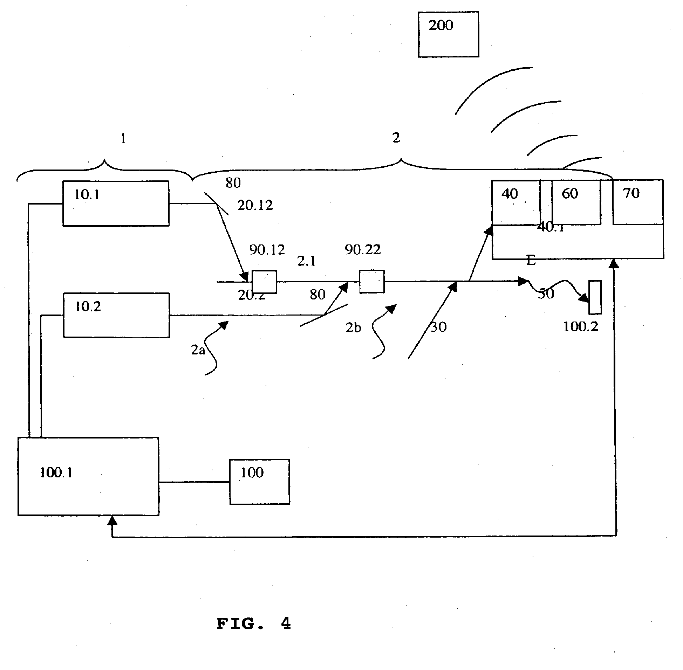

Weiter ist eine Leistungsüberwachung 40 vorhanden, wobei hier die Arbeitsstrahlen vollständig oder teilweise auskoppelbar sind und damit vor und während einer ophthalmologischen Anwendung festgestellt und geregelt werden kann, mit welcher Leistung die Arbeitsstrahlen 20.1 oder 20.2 durch die optische Faser 2.1 propagieren. Dabei ist es möglich, dass die Leistung der Arbeitsstrahlen 20.1 und 20.2 mittels Optimierung des Strahlengangs verbessert wird oder die Leistung der Arbeitsstrahlen 20.1 und 20.2 durch Ansteuerung der Laser 10.1 und 10.2 reduziert wird. Eine derartige Ansteuerung wird später in Fig. 4 näher beschrieben. Bevorzugt sind die Arbeitsstrahlen auch gesondert einkoppelbar.

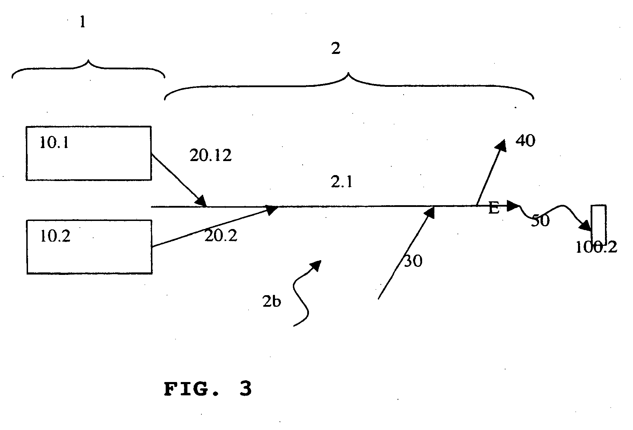

Die Figur 3 stellt eine weitere bevorzugte Ausführungsform des Multiwellenlängen-Lasersystems der vorliegenden Erfindung dar.

Dabei ist zusätzlich zu der in Figur 2 beschriebenen Anordnung des Multiwellenlängen-Lasersystems mit dem Lichtleitsystem am Ende E der optischen Faser 2.1 ein Lichtleitsystem mit einer Applikationsfaser 50 und einem Applikationssystem 100.2 angeschlossen, welche eine Schnittstelle zwischen der optischen Faser 2.1 und dem ophthalmologisch zu behandelnden Auge darstellt. Das Applikationssystem 100.2 umfasst in diesem Ausführungsbeispiel eine Spaltlampe, womit genauere Details des vorderen Teils des Auges gesehen werden können.

In der in Figur 3 darstellten Anordnung von Multiwellenlängen-Lasersystem 1 und Lichtleitsystem 2 werden zunächst die von den Halbleiter-Diodenlasern 10.1 und 10.2 emittierten Arbeitsstrahlen 20.1 und 20.2 in die optische Faser 2.1 des Lichtleitsystems 2 eingekoppelt, wobei das Lichtleitsystem 2 ein faseroptisches System 2b ist, und wobei die Arbeitsstrahlen 20.1 und 20.2 an gleichen oder verschiedenen Stellen der optischen Faser 2.1 eingekoppelt werden können.

Die eingekoppelten Arbeitsstrahlen 20.1 und 20.2 verlaufen weiter bei der Stelle in der optischen Faser 2.1 vorbei, wo später ein Pilotstrahl 30 zur Unterstützung der ophthalmologischen Anwendung eingekoppelt werden kann, laufen anschließend bei der Stelle in der optischen Faser 2.1 vorbei, wo eine Leistungsüberwachung 40 angeschlossen ist und wo die Energie und Leistung der Arbeitsstrahlen überwacht werden kann.

Nachdem die Arbeitsstrahlen 20.1 und 20.2 am Ende E der optischen Faser 2.1 angelangt sind, verlaufen sie direkt in die Applikationsfaser 50. Die Applikationsfaser 50 ist dabei derart angeschlossen, dass es zu keinen nennenswerten Energieund Leistungsverlusten der Arbeitsstrahlen 20.1 und 20.2 kommt.

Die Applikationsfaser 50 ist dabei für ophthalmologische Anwendungen in ihrer Dimensionierung und ihren Materialeigenschaften derart ausgelegt, dass die vorhergehend beschriebenen ophthalmologischen Anwendungen am Auge optimiert und anwendungsspezifisch durchgeführt werden können. Die Applikationsfaser 50 kann bevorzugt in das Auge eines Patienten eingeführt werden, damit ist eine entsprechende ophthalmologische Anwendung im Inneren des Auges möglich.

In Figur 4 ist Multiwellenlängen-Lasersystems 1 und Lichtleitsystems 2 dargestellt.

Dabei setzt sich das Multiwellenlängen-Lasersystem 1 aus den beiden Halbleiter-Diodenlasern 10.1 und 10.2, einer Nutzerschnittstelle 100 und einer Steuerung 100.1 zusammen. Die Halbleiter-Diodenlaser 10.1 und 10.2 sind, wie bereits in Fig. 1 beschrieben, ein InGaAlP-Laser und ein GaAlAs-Laser mit den angegebenen Parametern.

Das Lichtleitsystem 2 umfasst in dieser Ausführungsform neben der optischen Faser 2.1 ein mechanisches-optisches System 2a, welches sich aus den optischen Spiegeln 80, aber auch brechenden Optiken, zusammensetzt.

Weiter umfasst das Lichtleitsystem 2 ein faseroptisches System 2b, welches sich aus der optischen Faser 2.1, einer Dotierung 90.1, einem Polarisationskoppler 90.2 einer Einkopplung für einen Pilotstrahl 30 und einer Auskopplung für eine Leistungsüberwachung 40, einer Leistungssteuerung 40.1, einer Remote-Steuerung 60 oder einem System zur Auswertung rückgestreuter und/oder partiell reflektierter Arbeitsstrahlen 70 zusammensetzt. Darüber hinaus haben weitere teilnehmende Nutzer 200, wie beispielsweise weitere Ärzte, die Möglichkeit, eine ophthalmologische Anwendung online mitzuverfolgen und unterstützend bei einem ophthalmologischen Eingriff mitzuwirken.

Vor dem Ende E der optischen Faser 2.1 umfasst das Lichtleitsystem 2 ein Applikationssystem 100.2, in welchem sich eine Spaltlampe für ophthalmologische Anwendungen befindet. Darüber hinaus kann das Applikationssystem 100.2 ein Linksystem für den Anschluss eines weiteren Lasers zur Anwendung der selektiven Lasertrabekulotomie (SLT) umfassen. Ein derartiger Laser wird im pw-Modus betrieben und ist bevorzugt eine Laseranordnung aus aktiven Festkörpermaterialien und/ oder zusätzlichen Wellenlänge beeinflussenden optischen Elementen.

Die von den Halbleiter-Diodenlasern 10.1 und 10.2 emittierten Arbeitsstrahlen 20.1 und 20.2 durchlaufen zunächst ein mechanisch-optisches System, welches optische Spiegel 80 und optisch brechende Elemente aufweist. Die beiden Arbeitsstrahlen 20.1 und 20.2 werden dabei von den optischen Spiegeln 80 derart abgelenkt, dass sie an unterschiedlichen Stellen der optischen Faser 2.1 des Lichtleitsystems 2 eingekoppelt werden. Sie können aber ebenso mit nur einem optischen Spiegel 80 oder ohne Spiegel an gleichen oder unterschiedlichen Stellen der optischen Faser 2.1 eingekoppelt werden.

Anschließend durchläuft der erste Arbeitsstrahl 20.1 eine Dotierung 90.1 in der optischen Faser 2.1. Diese Dotierung 90.1 bewirkt, dass die Intensität und/oder die Wellenlänge der Arbeitsstrahlen modifiziert werden. Anschließend durchläuft der Arbeitsstrahl 20.1 einen Polarisationskoppler 90.2, welcher den Arbeitsstrahl 20.1 polarisiert.

Parallel dazu verläuft Arbeitsstrahl 20.2, welcher in dem hier gezeigten Beispiel am Spiegel 80 abgelenkt wird und bei einer späteren Position der optischen Faser 2.1 eingekoppelt wird. Arbeitsstrahl 20.2 durchläuft anschließend den Polarisationskoppler 90.2. Auch hier erfährt Arbeitsstrahl 20.2, so wie der Arbeitsstrahl 20.1, eine entsprechende selektive Polarisation.

Auch der Arbeitsstrahl 20.1 durchläuft den Polarisationskoppler 90.2.

Anschließend durchlaufen beide Arbeitsstrahlen, gleichzeitig oder zeitlich versetzt jene Stelle, wo vor einer ophthalmologischen Anwendung ein Pilotstrahl 30 eingekoppelt wird.

Zu einem späteren Zeitpunkt durchlaufen beiden Arbeitsstrahlen, gleichzeitig oder zeitlich versetzt jene Stelle, wo eine Leistungssteuerung 40.1, Leistungsüberwachung 40, eine Remote-Steuerung 60 oder ein System 70 zur Auswertung rückgestreuter und/oder reflektierter Arbeitsstrahlen angeschlossen werden.

Danach gelangen die Arbeitsstrahlen 20.1 und 20.2 zu einem Applikationssystem 100.2. In diesem befindet sich eine Spaltlampe, die zur Unterstützung der ophthalmologischen Anwendung dient, da damit während einer ophthalmologischen Anwendung gleichzeitig der Zustand des Auges überwacht wird.

Anschließend propagieren die Arbeitsstrahlen 20.1 und 20.2 am Ende E der optischen Faser 2.1 in die Applikationsfaser 50 und gelangen anschließend bei ophthalmologischer Anwendung direkt auf das zu behandelnde Auge.

Darüber hinaus wird erwähnt, dass die Leistungssteuerung 40.1, die Leistungsüberwachung 40, sowie das System zur Auswertung rückgestreuter und/oder reflektierter Arbeitsstrahlen mit der Steuerung 100.1 verbunden sind, und bei entsprechender Über- oder Unterschreitung der für die ophthalmologische Behandlung benötigten Werte (zu geringe/hohe Energie, zu hohe Leistung, zu starke Reflexion, ...) die Steuerung 100.1 zur Korrektur der emittierten Arbeitsstrahlen 20.1 oder 20.2 angesteuert wird und eine entsprechende Steuerungssoftware die Halbleiter-Diodenlaser 10.1 oder 10.2 selektiv ansteuert.

Die Remote-Steuerung 60 erlaubt darüber hinaus, Daten auf schnurlose Art und Weise oder mit Hilfe eines Netzwerkes oder des Internets an bei der ophthalmologischen Anwendung weitere teilnehmende Nutzer 200 zu übertragen. Somit ist es möglich, dass beispielsweise weitere Ärzte an der ophthalmologischen Behandlung teilnehmen können und entsprechende Empfehlungen abgeben können und somit beratend zur Seite stehen.

Darüber hinaus ist es mittels der Nutzerschnittstelle 100 möglich, dass ein Nutzer des Multiwellenlängen-Lasersystems 1 jederzeit manuell die Halbleiter-Diodenlaser 10.1 und 10.2 ansteuern kann und so die entsprechenden gewünschten Parameter für die ophthalmologische Anwendung einstellen kann.

Außerdem ist es einem Nutzer über die Nutzerschnittstelle 100 jederzeit möglich, das Multiwellenlängen-Lasersystem im Notfall auszuschalten und so eventuell auftretende Komplikationen während einer ophthalmologischen Anwendung zu verhindern.

Mit der erfindungsgemäßen Vorrichtung und dem Verfahren eines Multiwellenlängen-Lasersystems für ophthalmologische Anwendungen wurde ein Multiwellenlängen-Lasersystem bereitgestellt, welches kompakter und einfacher zu handhaben ist als Multiwellenlängen-Lasersysteme des Standes der Technik und welches die gleichzeitige, zeitlich verschobene oder räumlich verschobene Applikation mehrerer Wellenlängen aber auch unterschiedlicher zeitlicher Regime der Arbeitsstrahlen für die ophthalmologische Lasertherapie erlaubt.

### Bezugszeichenliste

- λ₁: erste Wellenlänge
- λ₂: zweite Wellenlänge
- 1: Multiwellenlängen-Lasersystem
- 2: Lichtleitsystem
- 2.1: optische Faser
- 2a: mechanisch-optisches System
- 2b: faseroptisches System
- 10.1: erster Halbleiter-Diodenlaser
- 10.2: zweiter Halbleiter-Diodenlaser
- 20.1: erster Arbeitsstrahl
- 20.2: zweiter Arbeitsstrahl
- 30: Pilotstrahl
- 40: Leistungsüberwachung
- 40.1: Leistungssteuerung
- 50: Applikationsfaser
- 60: Remote-Steuerung
- 70: System zur Auswertung rückgestreuter und/oder reflektierer Arbeitsstrahlen
- 80: optischer Spiegel
- 90.1: Dotierung
- 90.2: Polarisationskoppler
- 100: Nutzer-Schnittstelle
- 100.1: Steuerung
- 100.2: Applikationssystem (z.B. Spaltlampe)
- 200: Weiterer Nutzer

## Patentansprüche

1. Ophtalmologisches Multiwellenlängen-Lasersystem (1) mit einem Lichtleitsystem (2), umfassend:
mindestens ein Multiwellenlängen-Lasersystem (1), umfassend:
einen ersten Halbleiter-Diodenlaser (10.1) mit einem ersten Arbeitsstrahl (20.1), der eine erste Wellenlänge λ₁ aufweist, und
mindestens einen zweiten Halbleiter-Diodenlaser (10.2) mit einem zweiten Arbeitsstrahl (20.2), der eine zweite wellenlänge λ₂ aufweist,
wobei das Lichtleitsystem (2) ein faseroptisches System (2b) ist, das mindestens zwei optische Fasern umfasst, in denen die Arbeitsstrahlen (20.1, 20.2) geführt sind, die zu einer optischen Faser gekoppelt sind,
wobei die Arbeitsstrahlen (20.1, 20.2) des Multiwellenlängen-Lasersystems (1) an gleichen oder unterschiedlichen Stellen der optischen Faser des faseroptischen Systems in das Lichtleitsystem (2) eingekoppelt sind und wobei dadurch der Strahlengang der Arbeitsstrahlen (20.1, 20.2) nicht frei liegt und die Arbeitsstrahlen bis an ein Ende der optischen Faser propagierbar sind.

2. Ophtalmologisches Multiwellenlängen-Lasersystem (1) mit einem Lichtleitsystem (2) gemäß Anspruch 1, **dadurch gekennzeichnet, dass** der erste Arbeitsstrahl (20.1) eine erste Pulsdauer t₁ und der zweite Arbeitsstrahl (20.2) eine zweite Pulsdauer t₂ aufweist.

3. Ophtalmologisches Multiwellenlängen-Lasersystem (1) mit einem Lichtleitsystem (2) gemäß einem der vorhergehenden Ansprüchen, **dadurch gekennzeichnet, dass** ein Halbleiter-Diodenlaser einen Kristall umfasst aus der Gruppe: GaInAsSb, AlGaAsSb, AlAs, GaAs, GaP, InAs, AlGaInP, AlGaInP/GaAs, InP, dotierte Granate oder dotierte Vanadate.

4. Ophtalmologisches Multiwellenlängen-Lasersystem (1) mit einem Lichtleitsystem (2) gemäß einem der vorhergehenden Ansprüchen, **dadurch gekennzeichnet, dass** die Wellenlängen λ₁, λ₂ der Arbeitsstrahlen (20.1, 20.2) in einem der folgenden Spektralbereiche liegen: 480 nm bis 520 nm, 530 nm bis 550 nm, 560 nm bis 580 nm, 585 nm bis 615 nm, 620 nm bis 670 nm, 685 nm bis 695 nm, 800 nm bis 820 nm, 1040 nm bis 1070 nm.

5. Ophtalmologisches Multiwellenlängen-Lasersystem (1) mit einem Lichtleitsystem (2) gemäß einem der vorhergehenden Ansprüchen, **dadurch gekennzeichnet, dass** die Arbeitsstrahlen (20.1, 20.2) voneinander unterschiedliche Energien aufweisen.

6. Ophtalmologisches Multiwellenlängen-Lasersystem (1) mit einem Lichtleitsystem (2) gemäß einem der vorhergehenden Ansprüchen, **dadurch gekennzeichnet, dass** die Arbeitsstrahlen (20.1, 20.2) im cw-Modus (continuous wave mode) und/oder im pw-Modus(pulsed wave mode) emittiert werden.

7. Ophtalmologisches Multiwellenlängen-Lasersystem (1) mit einem Lichtleitsystem (2) gemäß einem der vorhergehenden Ansprüchen, **dadurch gekennzeichnet, dass** die Arbeitsstrahlen (20.1, 20.2) der Halbleiter-Diodenlaser (10.1, 10.2) getrennt voneinander steuerbar sind.

8. Ophtalmologisches Multiwellenlängen-Lasersystem (1) mit einem Lichtleitsystem (2) gemäß einem der vorhergehenden Ansprüchen, **dadurch gekennzeichnet, dass** das faseroptische System (2b), mindestens eine optische Faser (2.1) umfasst.

9. Ophtalmologisches Multiwellenlängen-Lasersystem (1) mit einem Lichtleitsystem (2) gemäß den vorhergehenden Ansprüchen, **dadurch gekennzeichnet, dass** an eine oder mehrere Fasern des faseroptischen Systems (2b) eine Applikationsfaser (50) anschließbar ist.

10. Ophtalmologisches Multiwellenlängen-Lasersystem (1) mit einem Lichtleitsystem (2) gemäß den vorhergehenden Ansprüchen, **dadurch gekennzeichnet, dass** eine numerische Apertur (N.A.) des Faserkerns des faseroptischen Systems (2b) zwischen 0,01 und 0,4, bevorzugt zwischen 0,05 und 0,22, besonders bevorzugt zwischen 0,07 und 0,15 beträgt.

11. Ophtalmologisches Multiwellenlängen-Lasersystem (1) mit einem Lichtleitsystem (2) gemäß den vorhergehenden Ansprüchen, **dadurch gekennzeichnet, dass** ein Pilotstrahl (30) in das Lichtleitsystem (2) einkoppelbar ist.

12. Ophtalmologisches Multiwellenlängen-Lasersystem (1) mit einem Lichtleitsystem (2) gemäß den vorhergehenden Ansprüchen, **dadurch gekennzeichnet, dass** die in das Lichtleitsystem (2) einkoppelbaren Arbeitsstrahlen (20.1, 20.2) zumindest teilweise auskoppelbar sind, insbesondere in eine Leistungsüberwachung (40) einkoppelbar sind.

13. Ophtalmologisches Multiwellenlängen-Lasersystem (1) mit einem Lichtleitsystem (2) gemäß einem der vorhergehenden Ansprüchen, **dadurch gekennzeichnet, dass** die Leistung und/oder Energie des ersten Halbleiter-Diodenlasers (10.1) und/oder des zweiten Halbleiter-Diodenlasers (10.2) mittels einer Leistungssteuerung (40.1) über eine Steuerung (100.1) einstellbar sind.

14. Verfahren für ophthalmologische Anwendungen, insbesondere selektiver Laser-Trabekulotomie (SLT) und/oder Laser-Trabekulotomie (ALT, DLT, LT) und/oder Laserkoagulation, wobei ein ophtalmologisches Multiwellenlängen-Lasersystem (1) gemäß den Ansprüchen 1 bis 13 verwendet werden.

## Claims

1. Ophthalmological multi-wavelength laser system (1) with an optical waveguide system (2), comprising:
at least one multi-wavelength laser system (1), comprising:
a first semiconductor diode laser (10.1) with a first working beam (20.1) which has a first wavelength λ₁ and
at least one second semiconductor diode laser (10.2) with a second working beam (20.2) which has a second wavelength λ₂,
wherein the optical waveguide system (2) is a fibre-optic system (2b) which comprises at least two optical fibres in which the working beams (20.1, 20.2) are guided, which are coupled to an optical fibre,
wherein the working beams (20.1, 20.2) of the multi-wavelength laser system (1) are coupled into the optical waveguide system (2) at the same or at different points of the optical fibre of the fibre-optic system and
wherein the beam path of the working beams (20.1, 20.2) is thereby not exposed and the working beams can be propagated up to one end of the optical fibre.

2. Ophthalmological multi-wavelength laser system (1) with an optical waveguide system (2) according to claim 1, **characterized in that** the first working beam (20.1) has a first pulse duration t₁ and the second working beam (20.2) has a second pulse duration t₂.

3. Ophthalmological multi-wavelength laser system (1) with an optical waveguide system (2) according to one of the previous claims, **characterized in that** a semiconductor diode laser comprises a crystal from the group: GaInAsSb, AlGaAsSb, AlAs, GaAs, GaP, InAs, AlGaInP, AlGaInP/GaAs, InP, doped garnets or doped vanadates.

4. Ophthalmological multi-wavelength laser system (1) with an optical waveguide system (2) according to one of the previous claims, **characterized in that** the wavelengths λ₁, λ₂ of the working beams (20.1, 20.2) lie in one of the following spectral ranges: 480 nm to 520 nm, 530 nm to 550 nm, 560 nm to 580 nm, 585 nm to 615 nm, 620 nm to 670 nm, 685 nm to 695 nm, 800 nm to 820 nm, 1040 nm to 1070 nm.

5. Ophthalmological multi-wavelength laser system (1) with an optical waveguide system (2) according to one of the previous claims, **characterized in that** the working beams (20.1, 20.2) have different energies from each other.

6. Ophthalmological multi-wavelength laser system (1) with an optical waveguide system (2) according to one of the previous claims, **characterized in that** the working beams (20.1, 20.2) are emitted in CW mode (continuous wave mode) and/or in PW mode (pulsed wave mode).

7. Ophthalmological multi-wavelength laser system (1) with an optical waveguide system (2) according to one of the previous claims, **characterized in that** the working beams (20.1, 20.2) of the semiconductor diode laser (10.1, 10.2) can be controlled separately from each other.

8. Ophthalmological multi-wavelength laser system (1) with an optical waveguide system (2) according to one of the previous claims, **characterized in that** the fibre-optic system (2b) comprises at least one optical fibre (2.1).

9. Ophthalmological multi-wavelength laser system (1) with an optical waveguide system (2) according to the previous claims, **characterized in that** an application fibre (50) can be connected to one or more fibres of the fibre-optic system (2b).

10. Ophthalmological multi-wavelength laser system (1) with an optical waveguide system (2) according to the previous claims, **characterized in that** a numerical aperture (NA) of the fibre core of the fibre-optic system (2b) is between 0.01 and 0.4, preferably between 0.05 and 0.22, particularly preferably between 0.07 and 0.15.

11. Ophthalmological multi-wavelength laser system (1) with an optical waveguide system (2) according to the previous claims, **characterized in that** a pilot beam (30) can be coupled into the optical waveguide system (2).

12. Ophthalmological multi-wavelength laser system (1) with an optical waveguide system (2) according to the previous claims, **characterized in that** the working beams (20.1, 20.2) that can be coupled into the optical waveguide system (2) can be at least partially coupled out, in particular can be coupled into a power control (40).

13. Ophthalmological multi-wavelength laser system (1) with an optical waveguide system (2) according to one of the previous claims, **characterized in that** the power and/or energy of the first semiconductor diode laser (10.1) and/or of the second semiconductor diode laser (10.2) can be set by means of a power control system (40.1) via a control (100.1).

14. Method for ophthalmological applications, in particular selective laser trabeculotomy (SLT) and/or laser trabeculotomy (ALT, DLT, LT) and/or laser coagulation, wherein an ophthalmological multi-wavelength laser system (1) according to claims 1 to 13 is used.

## Revendications

1. Système laser ophtalmologique à longueurs d'onde multiples (1) avec un système de conduction de lumière (2), comprenant :
au moins un système laser à longueurs d'onde multiples (1), comprenant :
un premier laser à diode semi-conductrice (10.1) avec un premier rayon de travail (20.1) présentant une première longueur d'onde λ₁ ; et
au moins un deuxième laser à diode semi-conductrice (10.2) avec un deuxième rayon de travail (20.2) présentant une deuxième longueur d'onde λ₂ ;
le système de conduction de lumière (2) étant un système de fibre optique (2b) comprenant au moins deux fibres optiques dans lesquelles les rayons de travail (20.1, 20.2) sont conduits et couplés à une fibre optique ;
les rayons de travail (20.1, 20.2) du système laser à longueurs d'onde multiples (1) étant couplés aux mêmes positions de la fibre optique du système de fibre optique ou dans des positions différentes dans le système de conduction de lumière (2) et le trajet de rayon des rayons de travail (20.1, 20.2) n'étant ainsi pas libre et les rayons de travail pouvant être propagés jusqu'à une extrémité de la fibre optique.

2. Système laser ophtalmologique à longueurs d'onde multiples (1) avec un système de conduction de lumière (2) selon la revendication 1, **caractérisé en ce que** le premier rayon de travail (20.1) présente une première durée d'impulsion t₁ et que le deuxième rayon de travail (20.2) présente une deuxième durée d'impulsion t₂.

3. Système laser ophtalmologique à longueurs d'onde multiples (1) avec un système de conduction de lumière (2) selon l'une quelconque des revendications précédentes, **caractérisé en ce qu'**un laser à diode semi-conductrice comprend un cristal composé du groupe : GaInAsSb, AlGaAsSb, AlAs, GaAs, GaP, InAs, AlGaInP, AlGaInP/GaAs, InP, le grenat doté ou le vanadate doté.

4. Système laser ophtalmologique à longueurs d'onde multiples (1) avec un système de conduction de lumière (2) selon l'une quelconque des revendications précédentes, **caractérisé en ce que** les longueurs d'onde λ₁, λ₂ des rayons de travail (20.1, 20.2) se situent dans l'une des plages spectrales suivantes : 480 nm à 520 nm, 530 nm à 550 nm, 560 nm à 580 nm, 585 nm à 615 nm, 620 nm à 670 nm, 685 nm à 695 nm, 800 nm à 820 nm, 1040 nm à 1070 nm.

5. Système laser ophtalmologique à longueurs d'onde multiples (1) avec un système de conduction de lumière (2) selon l'une quelconque des revendications précédentes, **caractérisé en ce que** les rayons de travail (20.1, 20.2) présentent des énergies différentes les unes des autres.

6. Système laser ophtalmologique à longueurs d'onde multiples (1) avec un système de conduction de lumière (2) selon l'une quelconque des revendications précédentes, **caractérisé en ce que** les rayons de travail (20.1, 20.2) sont émis en mode cw (en anglais « continuous wave mode », mode d'onde continue) et/ou en mode pw (en anglais « pulsed wave mode », mode d'onde pulsée).

7. Système laser ophtalmologique à longueurs d'onde multiples (1) avec un système de conduction de lumière (2) selon l'une quelconque des revendications précédentes, **caractérisé en ce que** les rayons de travail (20.1, 20.2) des lasers à diode semi-conductrice (10.1, 10.2) peuvent être commandés séparément les uns des autres.

8. Système laser ophtalmologique à longueurs d'onde multiples (1) avec un système de conduction de lumière (2) selon l'une quelconque des revendications précédentes, **caractérisé en ce que** le système de fibre optique (2b) comprend au moins une fibre optique (2.1).

9. Système laser ophtalmologique à longueurs d'onde multiples (1) avec un système de conduction de lumière (2) selon les revendications précédentes, **caractérisé en ce qu'**une fibre d'application (50) peut être rattachée à une ou plusieurs fibres du système de fibre optique (2b).

10. Système laser ophtalmologique à longueurs d'onde multiples (1) avec un système de conduction de lumière (2) selon les revendications précédentes, **caractérisé en ce qu'**une ouverture numérique (N.A.) des fibres du système de fibre optique (2b) se situe entre 0,01 et 0,4, de façon préférée entre 0,05 et 0,22, de façon particulièrement préférée entre 0,07 et 0,15.

11. Système laser ophtalmologique à longueurs d'onde multiples (1) avec un système de conduction de lumière (2) selon les revendications précédentes, **caractérisé en ce qu'**un rayon pilote (30) peut être couplé dans le système de conduction de lumière (2).

12. Système laser ophtalmologique à longueurs d'onde multiples (1) avec un système de conduction de lumière (2) selon les revendications précédentes, **caractérisé en ce que** les rayons de travail (20.1, 20.2) pouvant être couplés dans le système de conduction de lumière (2) peuvent être au moins en partie découplés, notamment être couplés dans une surveillance de puissance (40).

13. Système laser ophtalmologique à longueurs d'onde multiples (1) avec un système de conduction de lumière (2) selon l'une quelconque des revendications précédentes, **caractérisé en ce que** la puissance et/ou l'énergie du premier laser à diode semi-conductrice (10.1) et/ou le deuxième laser à diode semi-conductrice (10.2) sont réglables au moyen d'un élément de commande de puissance (40.1) via un élément de commande (100.1).

14. Procédé pour des applications ophtalmologiques, notamment trabéculectomie sélective au laser (SLT) et/ou trabéculectomie au laser (ALT, DLT, LT) et/ou coagulation par laser, un système laser ophtalmologique à longueurs d'onde multiples (1) selon les revendications 1 à 13 étant utilisé.
